# EUROPÄISCHE PATENTSCHRIFT

(11) **EP 2 192 958 B1**
(45) Veröffentlichungstag und Bekanntmachung des Hinweises auf die Patenterteilung: **28.08.2013**
(21) Anmeldenummer: 08774107.0
(22) Anmeldetag: 17.06.2008
(51) Int. Cl.: A61Q 5/08, A61Q 5/10, A61K 8/36, A61K 8/22, A61Q 5/04, A61Q 5/12

(54) **OXIDATIVE HAARBEHANDLUNG MIT VERRINGERTER HAARSCHÄDIGUNG**
OXIDATIVE HAIR TREATMENT WITH REDUCED DAMAGE TO HAIR
TRAITEMENT CAPILLAIRE OXYDANT AVEC ALTÉRATION RÉDUITE DES CHEVEUX

(30) Priorität: 05.10.2007 DE 102007048140
(43) Veröffentlichungstag der Anmeldung: 09.06.2010
(73) Patentinhaber: Henkel AG & Co. KGaA, 40589 Düsseldorf (DE)
(72) Erfinder: DÖRING, Thomas, AZ 35255 Scottsdale (US); BROCKMANN, Claudia, 40627 Düsseldorf (DE); PAULI, Kristin, 42119 Wuppertal (DE); HEYER, Michael, 40699 Erkrath (DE)
(86) Internationale Anmeldenummer: PCT/EP2008/057597
(87) Internationale Veröffentlichungsnummer: WO 2009/047022

(56) Entgegenhaltungen:
- EP-A- 1 419 761
- EP-A- 1 752 138
- DE-C- 839 991
- US-A1- 2003 233 714
- US-A1- 2004 205 904
- FERNANDO T ET AL: "A comparison of the fatty acids and sterols of seeds of weedy and vegetable species of Amaranthus spp", JOURNAL OF THE AMERICAN OIL CHEMISTS' SOCIETY, SPRINGER, BERLIN, DE, vol. 62, no. 1, 1 January 1985 (1985-01-01), pages 89-91, XP002552929, ISSN: 0003-021X, DOI: 10.1007/BF02541498
- DATABASE BIOSIS [Online] BIOSCIENCES INFORMATION SERVICE, PHILADELPHIA, PA, US 1988 AYORINDE F O ET AL: 'RAPID TRANSESTERIFICATION AND MASS SPECTROMETRIC APPROACH TO SEED OIL ANALYSIS' Database accession no. PREV198886052553

## Beschreibung

Die vorliegende Erfindung betrifft die kosmetische Verwendung eines Gemisches aus Linolsäure und Linolensäure während der oxidativen Haarbehandlung zur Verringerung der Haarschädigung und zur Verbesserung des Kopfhautempfindens. Ein entsprechendes Verfahren zur oxidativen Haarbehandlung, sowie die darin verwendbaren Mittel sind ebenso Gegenstand der vorliegenden Erfindung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Die permanente, oxidative Färbung sowie die Haarbleiche und die Fixierung im Rahmen der Dauerwelle finden meist als oxidative Haarbehandlung in Gegenwart von Oxidationsmitteln, wie Wasserstoffperoxyd, statt. Das Oxidationsmittel erzielt hierbei leider nicht nur die erwünschte kosmetische Wirkung, sondern schädigt zusätzlich die Struktur des Haarkeratins. Das keratineigene Cystin oxidiert dabei zur Cysteinsäure, was sich negativ auf die Stabilität, die Haptik und die Optik der Haarfaser auswirkt. Derart geschädigtes Haar wirkt stumpf und spröde. In extremen Fällen kommt es sogar zum Haarbruch.

Darüber hinaus kommt während der oxidativen Haarbehandlung die Kopfhaut mit dem Oxidationsmittel in Berührung. Aus diesem Grunde spürt der empfindliche Proband während der oxidativen Haarbehandlung oftmals ein Missempfinden auf der Kopfhaut. Dieses Missempfinden wird meist als Stechen oder Jucken beschrieben.

Das deutsche Gebrauchsmuster DE-U-299 18 993 betrifft Haarwuchsmittel, die eine Mischung aus Fettsäuren, Vitaminen und Bindemittel enthalten.

Die Patentanmeldung DE-A-41 13 346 hat wässrige Zusammensetzungen für die Haarkräftigung und zur Förderung des Haarwuchses zum Gegenstand, welche Ethanol, Phospholipide und Öle und/oder Fette enthalten.

Die Patentanmeldung WO-A1-2007/096045 betrifft hautberuhigende oxidative Haarbehandlungsmittel, die Linolsäure enthalten.

Die Druckschrift EP-A2-938 888 betrifft zweiteilige Zusammensetzungen, deren erstes Mittel einen pH-Wert von wenigstens pH 7 aufweist einen niederen Alkohol, ein Carboxyvinyl-Polymer und eine höhere Fettsäure enthält. Die zweite Zusammensetzung kann ein Oxidationsmittel enthalten. Das zweiteilige Mittel kann als Haarbleichmittel oder Haarfärbemittel ausgestaltet sein.

US 2003/0233714 offenbart zweiteilige oxidative Färbemittel, weiche einen Oxidationsfarbstoff und einem Oxidationsmittel enthalten, wobei eine Komponente zusätzlich ein Polyaikylenglycol(n)alkylamin sowie einen festen Fettstoff enthält. Das Färbemittel kann zusätzlich eine organische Säure ausgewählt aus Ölsäure, Linolsäure, Linolensäure und Mischungen davon enthalten. Die organischen Säuren werden als Hilfskomponente eingesetzt, um die konditionierende Verbindung Polyalkylenglycol(n)alkylamine zu protonieren und auf diese Weise deren Affinität zum Haar zu steigern.

Es ist die Aufgabe der vorliegenden Erfindung, die zuvor beschriebenen Nebenwirkungen oxidativer Haarbehandlungen bereits während der oxidativen Haarbehandlung zu verringern, ohne den Wirkungsgrad des oxidativen Kosmetikums, insbesondere bezüglich Farbintensität, Farbechtheit, Aufhellleistung bzw. Wellwirkung, zu verschlechtern.

Es wurde nun überraschenderweise gefunden, daß die Aufgabe in hervorragendem Maße durch die Verwendung eines Gemisches aus Linolsäure und Linolensäure gelöst wird. Die Wirkung entfaltet sich während der oxidativen Haarbehandlung.

Ein erster Gegenstand der vorliegenden Erfindung ist daher die kosmetische Verwendung eines Gemisches aus Linolsäure und Linolensäure zur Verminderung der oxidativen Haarschädigung im Rahmen einer oxidativen Haarbehandlung.

Insbesondere wird durch den Einsatz des erfindungsgemäßen Gemisches
- die Elastizität der Haarfaser,
- die Reißfestigkeit der Haarfaser
im Rahmen einer oxidativen Haarbehandlung verbessert, bzw. die durch eine oxidative Haarbehandlung hervorgerufene Verschlecheterung der vorgenannten Parameter verringert.

Unter einer oxidativen Haarbehandlung wird erfindungsgemäß die Einwirkung eines oxidativen kosmetischen Mittels, enthaltend in einem kosmetischen Träger mindestens ein Oxidationsmittel, auf Haar definiert.

Die Oxidationsmittel im Sinne der Erfindung sind von Luftsauerstoff verschieden und besitzen ein solches Oxidationspotenzial, das es ermöglicht, Disulfidbrücken innerhalb oder zwischen den Proteinen des Haarkeratins zu knüpfen, das natürliche Farbpigment Melanin oxidativ aufzuhellen und/oder ein Oxidationsfarbstoffvorprodukt vom Entwicklertyp zu oxidieren.

Als Oxidationsmittel kommt bevorzugt Wasserstoffperoxyd und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·nH₂O₂ (n ist eine positive ganze Zahl größer 0), Harnstoffperoxid und Melaminperoxid in Frage.

Erfindungsgemäß kann das oxidative kosmetische Mittel auch zusammen mit einem Katalysator auf das Haar aufgebracht werden, der die Oxidation des Substrats, wie beispielsweise Oxidationsfarbstoffvorprodukte oder Melanin, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung einer Färbung beschleunigt als auch die Farbnuance gezielt beeinflusst werden.

Das Oxidationsmittel ist bevorzugt in einer Menge von 1,0 bis 10 Gew.-%, insbesondere von 3,0 bis 10,0 Gew.-%, jeweils bezogen auf das Gewicht des anwendungsbereiten Mittels, in dem oxidativen kosmetischen Mittel enthalten.

Die erfindungsgemäße Kombination von Linolsäure und Linolensäure, sowie das Oxidationsmittel werden jeweils bevorzugt in einem kosmetischen Träger verwendet. Als kosmetische Träger eignen sich erfindungsgemäß besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bei einer gleichzeitigen Anwendung des oxidativen kosmetischen Mittels und einem kosmetischen Mittel, enthaltend eine Kombination aus Linolsäure und Linolensäure, können beide Mittel, gegebenenfalls erst unmittelbar vor der Anwendung, miteinander gemischt werden, oder nacheinander ohne Zwischenspülschritt auf das Haar aufgebracht werden. In einer bevorzugten Ausführungsform enthält das erfindungsgemäß verwendete oxidative kosmetische Mittel daher zusätzlich eine Kombination aus Linolsäure und Linolensäure.

Als unmittelbar vor der oxidativen Haarbehandlung versteht sich im Sinne der Erfindung eine Anwendung, an die sich direkt die oxidative Haarbehandlung anschließt, wobei das Linolsäure- und Linolensäure-haltige Mittel zuvor vom Haar gespült oder bevorzugt auf dem Haar belassen wurde und das Haar bevorzugt noch naß ist.

Unter Linolsäure wird die (Z, Z)-9,12-Octadecadiensäure verstanden, die erfindungsgemäß in Form der Säure oder als dessen Salz mit einem physiologisch verträglichem Gegenion eingesetzt wird. Als physiologisch verträgliche Gegenionen der verwendbaren Salze eignen sich bevorzugt Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit der Linolsäure selbst, oder durch permanente Quaternisierung besagter organischer Amine gebildet. Beispiele dieser kationischen organischen Ammoniumverbindungen sind 2-Ammonioethanol und 2-Trimethylammonioethanol. Bevorzugte physiologisch verträgliche Gegenionen sind das Ammoniumion, das Alkalimetallion, das Erdalkalimetallion oder das Zinkions, besonders bevorzugte Gegenionen sind das Ammoniumion, das Natriumion, das Kaliumion, das Kalziumion, das Magnesiumion oder das Zinkion.

Die Linolsäure ist in dem anwendungsbereiten Mittel in einer Menge von 0,01 Gew.-% bis 10 Gew.-%, insbesondere von 0,1 Gew.-% bis 1 Gew.-%, enthalten, wobei die Mengen jeweils auf das Gewicht des anwendungsbereiten Mittels bezogen werden.

Unter Linolensäure wird erfindungsgemäß mindestens eine Säure oder dessen Salz mit einem physiologisch verträglichem Gegenion verstanden, die ausgewählt wird, aus der Gruppe alpha-Linolensäure [(all-Z)-9-9,12,15-Octadecatriensäure], gamma-Linolensäure [(all-Z)-6,9,12-Octadecatriensäure]. In einer besonders bevorzugten Ausführungsform handelt es sich bei der Linolensäure um alpha-Linolensäure und/oder ein Salz mit einem physiologisch verträglichem Gegenion. Als physiologisch verträgliche Gegenionen der verwendbaren Salze eignen sich bevorzugt Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit der Linolensäure selbst, oder durch permanente Quaternisierung besagter organischer Amine gebildet. Beispiele dieser kationischen organischen Ammoniumverbindungen sind 2-Ammonioethanol und 2-Trimethylammonioethanol. Bevorzugte physiologisch verträgliche Gegenionen sind das Ammoniumion, das Alkalimetallion, das Erdalkalimetallion oder das Zinkions, besonders bevorzugte Gegenionen sind das Ammoniumion, das Natriumion, das Kaliumion, das Kalziumion, das Magnesiumion oder das Zinkion.

Die Linolensäure, insbesondere die alpha-Linolensäure, ist in dem anwendungsbereiten Mittel in einer Menge von 0,0001 Gew.-% bis 1 Gew.-%, insbesondere von 0,01 Gew.-% bis 0,1 Gew.-%, enthalten, wobei die Mengen jeweils auf das Gewicht des anwendungsbereiten Mittels bezogen werden.

Der erfindungsgemäße Effekt tritt besonders ausgeprägt ein, wenn die Linolsäure und die Linolensäure gemeinsam in einem Gewichtsverhältnisbereich von Linolsäure zu Linolensäure von 1 zu 0.0005 bis 1 zu 0.1, insbesondere von 1 zu 0,001 bis 1 zu 0,05, angewendet wird.

Ein zweiter Gegenstand der Erfindung sind kosmetische Mittel, enthaltend in einem kosmetischen Träger eine Kombination aus
(i) mindestens einem Oxidationsmittel und
(ii) einem Gemisch aus Linolsäure und Linolensäure.

Bevorzugte kosmetische Träger sind die des ersten Erfindungsgegenstandes.

Entsprechend bevorzugte Oxidationsmittel wurden ebenfalls im Rahmen des ersten Erfindungsgegenstandes erwähnt.

Unter Linolsäure wird die (Z,Z)-9,12-Octadecadiensäure verstanden, die erfindungsgemäß in Form der Säure oder als dessen Salz mit einem physiologisch verträglichem Gegenion eingesetzt wird. Die bevorzugten physiologisch verträglichen Gegenionen sind die des ersten Erfindungsgegenstandes.

Die erfindungsgemäßen kosmetischen Mittel enthalten die Linolsäure in einer Menge von 0,01 Gew.-% bis 10 Gew.-%, insbesondere von 0,1 Gew.-% bis 1 Gew.-%, enthalten, wobei die Mengen jeweils auf das Gewicht des Mittels bezogen werden.

Unter Linolensäure wird erfindungsgemäß alpha-Linolensäure (all-Z)-9-9,12,15-Octadecatriensäure und/oder gamma-Linolensäure (all-Z)-6,9,12-Octadecatriensäure verstanden, die jeweils in Form der Säure oder als deren Salz mit einem physiologisch verträglichem Gegenion eingesetzt wird. In einer besonders bevorzugten Ausführungsform handelt es sich um alpha-Linolensäure bzw. deren Salz mit einem physiologisch veträglichem Gegenion. Die bevorzugten physiologisch verträglichen Gegenionen sind die des ersten Erfindungsgegenstandes.

Die Linolensäure ist in dem erfindungsgemäßen Mittel in einer Menge von 0,0001 Gew.-% bis 1 Gew.-%, insbesondere von 0,01 Gew.-% bis 0,1 Gew.-%, enthalten, wobei die Mengen jeweils auf das Gewicht des Mittels bezogen werden.

Es ist erfindungsgemäß besonders bevorzugt, wenn die Linolsäure und die Linolensäure in einem Gewichtsverhältnisbereich von Linolsäure zu Linolensäure von 1 zu 0.0005 bis 1 zu 0.1, insbesondere von 1 zu 0,001 bis 1 zu 0,05, in dem erfindungsgemäßen Mittel enthalten sind.

Es ist erfindungsgemäß bevorzugt, das erfindungsgemäße Mittel im Rahmen einer Farbveränderung der Haare, zu verwenden. Zu diesem Zweck enthalten die kosmetischen Mittel zusätzlich mindestens eine farbverändernde Komponente.

Die farbverändernde Komponente wird wiederum bevorzugt ausgewählt
(a) aus mindestens einem Oxidationsfarbstoffvorprodukt vom Typ der Entwicklerkomponenten und gegebenenfalls zusätzlich mindestens einer Kupplerkomponente
   und/oder
(b) aus Oxofarbstoffvorprodukten
   und/oder
(c) aus mindestens einem direktziehenden Farbstoff
   und/oder
(d) aus mindestens einer Vorstufe naturanaloger Farbstoffe
   und/oder
(e) aus mindestens einem Bleichverstärker.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Besonders bevorzugte p-Phenylendiamine sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxy-ethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylen-diamin, N-(β-Methoxyethyl)-p-phenylendiamin und 5,8-Diaminobenzo-1,4-dioxan sowie ihren physiologisch verträglichen Salzen. Erfindungsgemäß ganz besonders bevorzugte p-Phenylendiaminderivate sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.Bevorzugte zweikernige Entwicklerkomponenten sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetra-methylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze. Ganz besonders bevorzugte zweikernige Entwicklerkomponenten sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenyl-pyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(ß-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, 3,4,5-Triamino-pyrazol, 1-Methyl-3,4,5-triaminopyrazol, 3,5-Diamino-1-methyl-4-methylaminopyrazol und 3,5-Diamino-4-(ß-hydroxyethyl)amino-1-methylpyrazol.

Unter den Pyrazolo[1,5-a]pyrimidinen kann man insbesondere nennen: Pyrazolo[1,5-a]pyrimidin-3,7-diamin; 2,5-Dimethyl-pyrazolo[1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Amino-pyrazolo[1,5-a]pyrimidin-5-ol; 2-(3-Aminopyrazolo[1,5-a]pyrimidin-7-ylamino)ethanol; 2-(7-Amino-pyrazolo[1,5-a]pyrimidin-3-ylamino)ethanol; 2-[(3-Aminopyrazolo[1,5-a]pyrimidin-7-yl)-(2-hydroxy-ethyl)amino]-ethanol; 2-[(7-Aminopyrazolo[1,5-a]pyrimidin-3-yl)-(2-hydroxy-ethyl)amino]-ethanol; 5,6-Dimethylpyrazolo[1,5-a]pyrimidin-3,7-diamin; 2,6-Dimethylpyrazolo[1,5-a]pyrimidin-3,7-diamin; 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist.

Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxy-thanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}-amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin und die physiologisch verträglichen Salzen der vorgenannten Verbindungen.

Die erfindungsgemäßen, kosmetischen Mittel enthalten die Entwicklerkomponenten bevorzugt in einer Menge von 0,005 bis 10 Gew.-%, vorzugsweise von 0,1 bis 5 Gew.-%, jeweils bezogen auf das gesamte Mittel.

Die erfindungsgemäßen, kosmetischen Mittel enthalten die Kupplerkomponenten bevorzugt in einer Menge von 0,005 bis 10 Gew.-%, vorzugsweise von 0,1 bis 5 Gew.-%, jeweils bezogen auf das gesamte Mittel.

Das erfindungsgemäße Mittel kann als farbverändernde Komponente in Form der Oxofarbstoffvorprodukte mindestens eine Kombination, aus mindestens einer Verbindung der Komponente
1 Verbindungen, die eine reaktive Carbonylgruppe enthalten mit mindestens einer Verbindung der Komponente
2 Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen
enthalten.

Erfindungsgemäße Verbindungen mit einer reaktiven Carbonylgruppe (im Folgenden auch reaktive Carbonylverbindungen oder Komponente 1 genannt) besitzen mindestens eine Carbonylgruppe als reaktive Gruppe, welche mit den Verbindungen der Komponente 2 unter Ausbildung einer beide Komponenten verknüpfenden chemischen Bindung reagiert. Ferner sind erfindungsgemäß auch solche Verbindungen als Komponente 1 umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente 2 stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) Alkoholen unter Bildung von Acetalen oder Ketalen als Kondensationsverbindung
c) Wasser unter Bildung von Hydraten als Kondensationsverbindung von Aldehyden.

Als Vorstufen naturanaloger Farbstoffe werden bevorzugt als Oxidationsfarbstoffvorprodukt vom Entwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins. esonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Als Vorstufen naturanaloger Haarfarbstoffe hervorragend geeignet sind weiterhin Derivate des 5,6-Dihydroxyindols. Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Bevorzugte direktziehende Farbstoffe, die in den kosmetischen Mitteln als farbverändernde Komponente Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die kosmetischen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die kosmetischen Mittel enthalten die direktziehenden Farbstoffe bevorzugt in einer Menge von 0,01 bis 20 Gew.-%, bezogen auf das anwendungsbereite Mittel.

Weiterhin können die erfindungsgemäßen kosmetischen Mittel auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den kosmetischen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Das eigentliche oxidative Färbemittel wird bei getrennter Lagerung der Farbstoffvorprodukte und des Oxidationsmittels unmittelbar vor der Anwendung durch Mischen hergestellt. In einer bevorzugten Ausführungsform wird daher das kosmetische Mittel vor der Applikation aus einer Zusammensetzung, enthaltend in einem kosmetischen Träger mindestens eine farbverändernde Komponente, und einer weiteren Zusammensetzung, enthaltend in einem kosmetischen Träger mindestens ein Oxidationsmittel, gemischt.

Bei einer Anwendung von Oxidationsmitteln wird die anwendungsfertige Zubereitung zweckmäßigerweise unmittelbar vor der Anwendung durch Mischung einer Zusammensetzung, enthaltend das Oxidationsmittel mit der Zusammensetzung, enthaltend die farbverändernden Komponenten, hergestellt. Das dabei entstehende gebrauchsfertige Haarpräparat sollte bevorzugt einen pH-Wert im Bereich von 6 bis 12, insbesondere von pH 7,5 bis 10, aufweisen.

Für eine Farbveränderung mittels Aufhellung bzw. Bleichung der Haare wird bevorzugt in den erfindungsgemäßen kosmetischen Mitteln neben den Oxidationsmitteln zusätzlich mindestens ein Bleichverstärker eingesetzt.

Bleichverstärker werden bevorzugt in Blondiermitteln zur Steigerung der Blondierwirkung des Oxidationsmittels, insbesondere des Wasserstoffperoxyds, eingesetzt.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker können erfindungsgemäß bevorzugt Carbonatsalze, bzw. Hydrogencarbonatsalze eingesetzt werden. Diese sind bevorzugt ausgewählt aus der Gruppe der Ammonium-, Alkali- (insbesondere Natrium- und Kalium-), sowie Erdalkali- (insbesondere Calcium-), -carbonatsalze bzw. -hydrogencarbonatsalze. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (V), 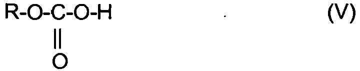 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (V) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (V) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Alternativ zum Kohlensäuremonoester oder in Verbindung mit ihm können in den wasserfreien Zusammensetzungen Kohlensäuremonoamide als Bleichverstärker eingesetzt werden. Hier ist es erfindungsgemäß bevorzugt, mindestens ein Kohlensäuremonoamid der Formel (VI) zu verwenden, 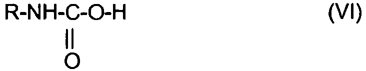 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (VI) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (VI) sind dadurch gekennzeichnet, daß der Rest R in Formel (VI) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet. Bevorzugt werden Silylcarbonate gemäß Formel (VII) eingesetzt, 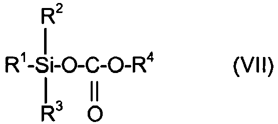 in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstiuierte Arylgruppe bzw. einen substituierten oder unsubstituierten Heterocyclus oder für ein Halogen, eine substituierte oder unsubstituierte Hydroxy-, Oxo-, Aminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste R¹, R² und R³ in der oben genannten Formel (VII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße wasserfreie Zusammensetzungen dadurch gekennzeichnet sind, daß die Reste R¹, R² und R³ in Formel (VII) ausgewählt sind aus Methyl-, Ethyl-, n-Propyl-, iso-Propyl-, n-Butyl-, iso-Butyl-, *tert-*Butyl- sowie Hydroxymethyl- und Hydroxyethyl-Resten.

Bevorzugte Reste R⁴ in der oben genannten Formel (VII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als Bleichverstärker kann mindestens ein Silylcarbamat der Formel (VIII) in der erfindungsgemäßen wasserfreien Zusammensetzung enthalten sein, 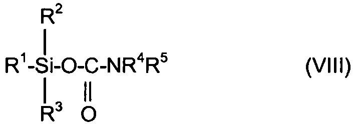 wobei die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste R¹, R² und R³ in der oben genannten Formel (VIII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste R¹, R² und R³ in Formel (VIII) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ und R⁵ in der oben genannten Formel (VIII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Bleichverstärker sind bevorzugt Peroxoverbindungen. Unter die erfindungsgemäß bleichverstärkenden Peroxoverbindungen fallen keine Anlagerungsprodukte von Wasserstoffperoxyd an andere Komponenten und auch nicht Wasserstoffperoxyd selbst. Die Auswahl der Peroxoverbindungen unterliegt darüber hinaus keinen Beschränkungen. Bevorzugte Peroxoverbindungen sind Peroxidisulfatsalze, Persulfatsalze, (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Bleichverstärker sind in den erfindungsgemäßen, kosmetischen Mitteln bevorzugt in Mengen von 5-30 Gew.-%, insbesondere in Mengen von 8-20 Gew.-%, enthalten.

Die kosmetischen Mittel der Erfindung enthalten, wenn sie als Bleichmittel fungieren, als bevorzugtes Alkalisierungsmittel mindestens eine Verbindung, ausgewählt aus Ammonium-, Alkalimetall- und Erdalkalimetallhydroxiden, -carbonaten, -hydrogencarbonaten, -hydroxycarbonaten, -metasilikaten und -carbamiden, sowie Alkaliphosphaten.

Die in dem erfindungsgemäßen Verfahren eingesetzten kosmetischen Mittel können weiterhin alle für solche Zubereitungen bekannten Wirk-, Zusatz- und Hilfsstoffe enthalten:

Das Nachwaschen mit einem Shampoo entfällt, wenn ein stark tensidhaltiger Träger verwendet wurde.

In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den kosmetischen Mitteln alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von ge-sättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Po-lyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Be-zeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltri-methylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die erfindungsgemäßen Mittel können zusätzlich mindestens ein Silikon enthalten. Die Silikone, wenn sie in den erfindungsgemäßen Mitteln zugegen sind, vorzugsweise in Mengen von 0,05 bis 5 Gew.-%, vorzugsweise von 0,2 bis 5 Gew.-%, jeweils bezogen auf das anwendungsbereite Mittel, enthalten.

Insbesondere bevorzugt werden die Silikone ausgewählt, aus mindestens einem Vertreter aus der Liste, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Selbstverständlich können auch Mischungen der o.g. Silikone in den bevorzugten erfindungsgemäßen Mitteln enthalten sein.

Bevorzugte erfindungsgemäß einsetzbare Silikone weisen bei 20°C Viskositäten von 0,2 bis 2 mm²S⁻¹ auf, wobei Silikone mit Viskositäten von 0,5 bis 1 mm²s⁻¹ besonders bevorzugt sind.

Bevorzugte erfindungsgemäße Mittel enthalten in einem kosmetischen Träger neben der Kombination aus (a) mindestens einem Oxidationsmittel (bevorzugt Wasserstoffperoxid) und (b) einem Gemisch aus Linolsäure und Linolensäure, zusätzlich eine der folgenden Wirkstoffkombinationen (i) bis (iii):
(i) mindestens eine farbverändernde Komponente und mindestens ein Silikon,
(ii) mindestens eine Entwicklerkomponente (und gegebenenfalls eine Kupplerkomponente) und mindestens ein Silikon und
(iii) mindestens einen Bleichverstärker und mindestens ein Silikon.

Zusätzlich können die erfindungsgemäßen Mittel mindestens ein Proteinhydrolysat enthalten. Die Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Keratin DEC^{®} (Vincience), Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben. Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich. Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben. Bevorzugt sind die Proteinhydrolysate in einer Menge von 0,05 bis 5 Gew.-%, besonders bevorzugt von 0,5 bis 2,0 Gew.-%, jeweils bezogen auf das Gewicht des anwendungsbereiten Mittels, enthalten.

Bevorzugte erfindungsgemäße Mittel enthalten in einem kosmetischen Träger neben der Kombination aus (a) mindestens einem Oxidationsmittel (bevorzugt Wasserstoffperoxid) und (b) einem Gemisch aus Linolsäure und Linolensäure, zusätzlich eine der folgenden Wirkstoffkombinationen (i) bis (xii):
(i) mindestens eine farbverändernde Komponente und mindestens ein Proteinhydrolysat,
(ii) mindestens eine farbverändernde Komponente und mindestens ein Weizenproteinhydrolysat,
(iii) mindestens eine farbverändernde Komponente und mindestens ein Seidenproteinhydrolysat,
(iv) mindestens eine farbverändernde Komponente und mindestens ein Kollagenproteinhydrolysat,
(v) mindestens eine Entwicklerkomponente (und gegebenenfalls eine Kupplerkomponente) und mindestens ein Proteinhydrolysat,
(vi) mindestens eine Entwicklerkomponente (und gegebenenfalls eine Kupplerkomponente) und mindestens ein Weizenproteinhydrolysat,
(vii) mindestens eine Entwicklerkomponente (und gegebenenfalls eine Kupplerkomponente) und mindestens ein Seidenproteinhydrolysat,
(viii) mindestens eine Entwicklerkomponente (und gegebenenfalls eine Kupplerkomponente) und mindestens ein Kollagenproteinhydrolysat,
(ix) mindestens einen Bleichverstärker und mindestens ein Proteinhydrolysat,
(x) mindestens einen Bleichverstärker und mindestens ein Weizenproteinhydrolysat,
(xi) mindestens einen Bleichverstärker und mindestens ein Seidenproteinhydrolysat,
(xii) mindestens einen Bleichverstärker und mindestens ein Kollagenproteinhydrolysat.

In einer weiteren Ausführungsform enthalten die erfindungsgemäßen Mittel zusätzlich mindestens ein kationisches und/oder mindestens ein amphoteres Polymer.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
   sowie die unter den Bezeichnungen
- Polyquaternium 2 (z.B. Mirapol® A-15 der Firma Rhodia),
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben. Besonders gut geeignete Chitosane weisen einen Deacetylierungsgrad von wenigstens 80 % und ein Molekulargewicht von 5 · 10⁵ bis 5 · 10⁶ (g/mol) auf.

Unter dem Begriff amphotere Polymere werden solche Polymere verstanden,
- die im Molekül sowohl freie Aminogruppen als auch freie -COOH- oder SO₃H-Gruppen enthalten und zur Ausbildung innerer Salze befähigt sind,
- zwitterionische Polymere, die im Molekül quartäre Ammoniumgruppen und -COO⁻-Gruppen oder -SO₃⁻-Gruppen enthalten, sowie
- Polymere, die -COOH-Gruppen oder SO₃H-Gruppen und quartäre Ammoniumgruppen enthalten.

Die in der Aufzählung genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben. Erfindungsgemäß ganz besonders bevorzugt verwendete amphotere Polymere auf Basis eines Comonomers sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Bezüglich der Einzelheiten der Herstellung dieser besonders bevorzugten Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (i) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (i). Als Monomeres (ii) für die genannten Polymerisate wird bevorzugt Acrylsäure verwendet.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Bevorzugte erfindungsgemäße Mittel enthalten in einem kosmetischen Träger neben der Kombination aus (a) mindestens einem Oxidationsmittel (bevorzugt Wasserstoffperoxid) und (b) einem Gemisch aus Linolsäure und Linolensäure, zusätzlich eine der folgenden Wirkstoffkombinationen (i) bis (vi):
(i) mindestens eine farbverändernde Komponente und mindestens ein kationisches Polymer,
(ii) mindestens eine farbverändernde Komponente und mindestens ein amphoteres Polymer,
(iii) mindestens eine Entwicklerkomponente (und gegebenenfalls eine Kupplerkomponente) und mindestens ein kationisches Polymer,
(iv) mindestens eine Entwicklerkomponente (und gegebenenfalls eine Kupplerkomponente) und mindestens ein amphoteres Polymer,
(v) mindestens einem Bleichverstärker und mindestens ein kationisches Polymer,
(vi) mindestens einem Bleichverstärker und mindestens ein amphoteres Polymer.

In einer weiteren bevorzugten Ausführungsform der Erfindung kann die Wirkung durch Zusatz von mindestens einem Fettstoff weiter optimiert werden. Unter Fettstoffen sind zu verstehen Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettalkohole können eingesetzt werden gesättigte, ein- oder mehrfach ungesättigte, verzweigte oder unverzweigte Fettalkohole mit C₆ - C₃₀-, bevorzugt C₁₀-C₂₂- und ganz besonders bevorzugt C₁₂-C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆-C₃₀-Fettsäuren mit C₂-C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, wie beispielsweise Monomuls^{®} 90-O18, Monomuls^{®} 90-L12 oder Cutina^{®} MD.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 6 - 45 Gew.%, bezogen auf das gesamte Mittel. Mengen von 10- 35 Gew.-% sind erfindungsgemäß bevorzugt.

Weiterhin hat sich gezeigt, daß die Wirkung gesteigert werden kann, wenn die erfindungsgemäßen Mittel zusätzlich mindestens einen Hydroxycarbonsäureester enthalten. Bevorzugte Hydroxycarbonsäureester sind Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Die Einsatzmenge der Hydroxycarbonsäureester beträgt dabei 0,1 - 15 Gew.% bezogen auf das Mittel, bevorzugt 0,1 - 10 Gew.% und ganz besonders bevorzugt 0,1 - 5 Gew.%.

Bevorzugte erfindungsgemäße Mittel enthalten in einem kosmetischen Träger neben der Kombination aus (a) mindestens einem Oxidationsmittel (bevorzugt Wasserstoffperoxid) und (b) einem Gemisch aus Linolsäure und Linolensäure, zusätzlich eine der folgenden Wirkstoffkombinationen (i) bis (xii):
(i) mindestens eine farbverändernde Komponente und mindestens einen Fettstoff,
(ii) mindestens eine farbverändernde Komponente und mindestens einen C₁₀ bis C₂₂-Fettalkohol,
(iii) mindestens eine farbverändernde Komponente und mindestens ein natürliches oder synthetisches Wachs,
(iv) mindestens eine farbverändernde Komponente und mindestens einen natürlichen oder synthetischen Ölkörper,
(v) mindestens Entwicklerkomponente (und gegebenenfalls eine Kupplerkomponente) und mindestens einen Fettstoff,
(vi) mindestens eine Entwicklerkomponente (und gegebenenfalls eine Kupplerkomponente) und mindestens einen C₁₀ bis C₂₂-Fettalkohol,
(vii) mindestens eine Entwicklerkomponente (und gegebenenfalls eine Kupplerkomponente) und mindestens ein natürliches oder synthetisches Wachs,
(viii) mindestens eine Entwicklerkomponente (und gegebenenfalls eine Kupplerkomponente) und mindestens einen natürlichen oder synthetischen Ölkörper,
(ix) mindestens einen Bleichverstärker und mindestens einen Fettstoff,
(x) mindestens einen Bleichverstärker und mindestens einen C₁₀ bis C₂₂-Fettalkohol,
(xi) mindestens einen Bleichverstärker und mindestens ein natürliches oder synthetisches Wachs,
(xii) mindestens einen Bleichverstärker und mindestens einen natürlichen oder synthetischen Ölkörper.

Es ist erfindungsgemäß bevorzugt, dass die Mittel zusätzlich mindestens ein Alkalisierungsmittel enthalten. Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxid, L-Arginin, D-Arginin, DL-Arginin, N-Methylglucamin, Morpholin, Imidazol und Harnstoff.

Bevorzugte erfindungsgemäße Mittel enthalten in einem kosmetischen Träger neben der Kombination aus (a) mindestens einem Oxidationsmittel (bevorzugt Wasserstoffperoxid) und (b) einem Gemisch aus Linolsäure und Linolensäure, zusätzlich eine der folgenden Wirkstoffkombinationen (i) bis (xii):
(i) mindestens eine farbverändernde Komponente und Ammoniak,
(ii) mindestens eine farbverändernde Komponente und mindestens ein Alkanolamin,
(iii) mindestens eine farbverändernde Komponente und 2-Aminoethan-1-ol,
(iv) mindestens eine farbverändernde Komponente und Ammoniak und 2-Aminoethan-1-ol,
(v) mindestens eine farbverändernde Komponente und 2-Aminoethan-1-ol und L-Arginin,
(vi) mindestens Entwicklerkomponente (und gegebenenfalls eine Kupplerkomponente) und Ammoniak,
(vii) mindestens eine Entwicklerkomponente (und gegebenenfalls eine Kupplerkomponente) und mindestens ein Alkanolamin,
(viii) mindestens eine Entwicklerkomponente (und gegebenenfalls eine Kupplerkomponente) und 2-Aminoethan-1-ol,
(ix) mindestens eine Entwicklerkomponente (und gegebenenfalls eine Kupplerkomponente) und Ammoniak und 2-Aminoethan-1-ol,
(x) mindestens eine Entwicklerkomponente (und gegebenenfalls eine Kupplerkomponente) und 2-Aminoethan-1-ol und L-Arginin,
(xi) mindestens einen Bleichverstärker und Ammoniak,
(xii) mindestens einen Bleichverstärker und mindestens ein Alkanolamin,
(xiii) mindestens einen Bleichverstärker und 2-Aminoethan-1-ol,
(xiv) mindestens einen Bleichverstärker und Ammoniak und 2-Aminoethan-1-ol,
(xv) mindestens einen Bleichverstärker und 2-Aminoethan-1-ol und L-Arginin.

Ferner können die kosmetischen Mittel weitere Wirk-, Hilfs- und Zusatzstoffe enthalten, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxyd und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Für den zweiten Erfindungsgegenstand gilt mutatis mutandis das für den ersten Erfindungsgegenstand Gesagte.

Ein dritter Erfindungsgegenstand ist ein Verfahren zur oxidativen Haarbehandlung, in dem ein oxidatives kosmetisches Mittel, enthaltend in einem kosmetischen Träger mindestens ein Oxidationsmittel auf das Haar aufgetragen wird und nach einer Einwirkungszeit vom Haar gespült wird, wobei
- unmittelbar vor dem Auftragen des oxidativen kosmetischen Mittels ein kosmetisches Mittel, enthaltend in einem kosmetischen Träger ein Gemisch aus Linolsäure und Linolensäure, aufgetragen und gegebenenfalls nach einer Einwirkungszeit wieder abgespült wird
   und/oder
- das oxidative kosmetische Mittel zusätzlich ein Gemisch aus Linolsäure und Linolensäure, enthält.

Die Einwirkungszeit des Gemischs aus Linolsäure und Linolensäure beträgt bevorzugt 1 bis 100 Minuten, besonders bevorzugt 5 bis 50 Minuten.

Es ist wiederum erfindungsgemäß bevorzugt das erfindungsgemäße Verfahren im Rahmen einer oxidativen Haarfärbung, der oxidativen Haarbleiche oder der Fixierung im Rahmen einer Dauerwelle anzuwenden. Dabei ist es bevorzugt, ein Gemisch aus Linolsäure und Linolensäure gemeinsam mit dem Oxidationsmittel auf dem Haar anzuwenden.

In einer bevorzugten Ausführungsform des erfindungsgemäßen Verfahrens als oxidatives Haarfärbeverfahren, wird bevorzugt ein Gemisch aus Linolsäure und Linolensäure als Bestandteil eines kosmetischen Mittels enthaltend in einem kosmetischen Träger zusätzlich mindestens ein Oxidationsmittel und mindestens ein Oxidationsfarbstoffvorprodukt in einem Schritt angewendet.

Die oxidativen Haarfärbemittel dieser Ausführungsform sind bevorzugt Zweikomponenten Mittel. Die erste Komponente enthält in einem kosmetischen Träger die ein Gemisch aus Linolsäure und Linolensäure und mindestens ein Oxidationsfarbstoffvorprodukt. Die zweite Komponente enthält in -einem kosmetischen Träger mindestens ein Oxidationsmittel. Diese Komponenten werden bevorzugt getrennt voneinander in jeweils einem Kompartiment konfektioniert und gemeinsam in einer Verpackungseinheit (Kit) bereitgestellt. Kurz vor der Anwendung werden beide Komponenten miteinander vermischt.

In der Ausführungsform des erfindungsgemäßen Verfahrens als oxidatives Haarbleichverfahren, wird bevorzugt die ein Gemisch aus Linolsäure und Linolensäure als Bestandteil eines kosmetischen Mittels enthaltend in einem kosmetischen Träger zusätzlich mindestens ein Oxidationsmittel und mindestens einen Bleichverstärker in einem Schritt angewendet.

Die oxidativen Haarbleichmittel dieser Ausführungsform sind bevorzugt Zwei- oder Dreikomponenten-Mittel. Die erste Komponente enthält in einem kosmetischen Träger die ein Gemisch aus Linolsäure und Linolensäure und gegebenenfalls mindestens einen Bleichverstärker. Der Bleichverstärker kann auch getrennt von dem Gemisch aus Linolsäure und Linolensäure konfektioniert sein, beispielsweise in Pulverform, als wasserfreie Paste oder wasserfreies Öl. Dadurch wird ein Dreikomponenten-Mittel erhalten. Die letzte Komponente enthält in einem kosmetischen Träger mindestens ein Oxidationsmittel. Alle Komponenten werden bevorzugt getrennt voneinander in jeweils einem Kompartiment konfektioniert und gemeinsam in einer Verpackungseinheit (Kit) bereitgestellt. Kurz vor der Anwendung werden alle Komponenten miteinander gemischt.

Die zuvor beschriebenen Mehrkomponentenmittel zur Durchführung des erfindungsgemäßen Verfahrens können in einer Verpackungseinheit bereitgestellt werden. Die Verpackungseinheit kann mindestens entweder einen Kontainer umfassen, der das Mittel des zweiten Erfindungsgegenstandes enthält, oder kann mindestens zwei Kontainer umfassen, wobei ein erster Kontainer ein oxidatives kosmetisches Mittel, enthaltend in einem kosmetischen Träger mindestens ein Oxidationsmittel, enthält, ein zweiter Kontainer ein kosmetische Mittel, enthaltend in einem kosmetischen Träger ein Gemisch aus Linolsäure und Linolensäure, enthält und gegebenenfalls ein dritter Kontainer, der mindestens einen Bleichverstärker, eingebettet in einem kosmetischen Träger, enthält. Alle Kontainer können auch Kammern eines Mehrkammerbehältnisses sein.

Für den dritten Erfindungsgegenstand gilt mutatis mutandis das für den ersten und zweiten Erfindungsgegenstand Gesagte.

Die folgenden Beispiele sollen den Gegenstand der vorliegenden Erfindung erläutern ohne ihn jedoch zu beschränken.

### Beispiele

Die folgenden Rezepturen wurden unter Anwendung bekannter Herstellungsverfahren bereitgestellt. Dabei wurden nachstehende Handelsprodukte als Rohstoffe verwendet:

| | |
|---|---|
| Hydrenol^{®} D | C₁₆-C₁₈-Fettalkohol (INCI-Bezeichnung: Cetearyl alcohol) (Cognis Deutschland) |
| Lorol^{®} techn. | C₁₂-C₁₈-Fettalkohol (INCI-Bezeichnung: Coconut alcohol) (Cognis Deutschland) |
| Stenol^{®} 16/18 | C₁₆₋₁₈-Fettalkohol (INCI-Bezeichnung: Cetearyl Alcohol) (Cognis) |
| Lorol^{®} 16 | INCI-Bezeichnung: Cetyl Alcohol (Cognis) |
| Eumulgin^{®} B1 | Cetylstearylakohol mit 12 EO-Einheiten (INCI-Bezeichnung: Ceteareth-12) (Cognis Deutschland) |
| Eumulgin^{®} B2 | Cetylstearylalkohol mit ca. 20 EO-Einheiten (INCI-Bezeichnung: Ceteareth-20) (Cognis Deutschland) |
| Edenor^{®} C14 | Myristinsäure (INCI-Bezeichnung: Myristic Acid) (Cognis) |
| Turpinal^{®} SL | 1-Hydroxyethan-1,1-diphosphonsäure (INCI-Bezeichnung: Etidronic Acid, Aqua (Water)) (Solutia) |
| Plantapon^{®} LGC | Alkylpolyglucosid-Carboxylat Natriumsalz; 30 % Aktivsubstanz (Cognis Deutschland) |
| Texapon^{®} NSO UP | Natriumlaurylethersulfat (27 % Aktivsubstanz; INCI: Sodium Laureth Sulfate) (Hersteller : COGNIS) |
| Texapon^{®} K 14 S 70 C | Laurylmyristylethersulfat-Natrium-Salz (ca. 68% bis 73% Aktivsubstanz gehalt; INCI-Bezeichnung: Sodium Myreth Sulfate) (Cognis) |
| Disponil^{®} FES 77 IS | Fettalkoholethersulfat (ca. 31-33% Aktivsubstanzgehalt in Wasser; INCI-Bezeichnung: Sodium Coceth-30 Sulfate) (Cognis) |
| Akypo^{®} Soft 45 NV | 2-(C₁₂₋₁₄-Fettalkoholethoxylat (4.5 EO))-essigsäure Natriumsalz; 21% Aktivsubstanz; INCI-Bezeichnung: Sodium Laureth-5 Carboxylate (KAO) |
| Gluadin^{®} W 40 | Weizenproteinhydrolysat (mind. 40% Festkörper; INCI-Bezeichnung: Aqua (Water), Hydrolyzed Wheat Protein, Sodium Benzoate, Phenoxyethanol, Methylparaben, Propylparaben) (Cognis) |
| Plantacare^{®} 1200 UP | C₁₂₋₁₆-Fettalkohol-1.4-glucosid (ca. 50-53% Aktivsubstanzgehalt; INCI-Bezeichnung: Lauryl Glucoside, Aqua (Water)) (Cognis) |
| Lamesoft^{®} PO 65 | Alkylpolyglucosid Ölsäuremonoglycerid Gemisch (ca. 65-70% Festkörper; INCI-Bezeichnung: Coco-Glucoside, Glyceryl Oleate, Aqua (Water)) (Cognis) |
| Aculyn^{®} 33 | 30 Gew.-% Aktivsubstanz in Wasser (INCI-Bezeichnung: Acrylates Copolymer) (Rohm & Haas) |
| DOW Corning^{®} DB 110 A | nichtionogene Silikonemulsion (10 Gew.-% Aktivsubstanz) (INCI-Bezeichnung: Dimethicone) (Dow Corning) |
| Aristoflex^{®} AVC | Copolymer von Vinylpyrrolidon und Ammoniumacryloyl-dimethyltaurat; (INCI-Bezeichnung: Ammonium Acryloyldimethyl-taurate/VP Copolymer, t-Butyl Alcohol) (Clariant) |
| Polymer^{®} W 37194 | ca. 20Gew.-% Aktivsubstanzgehalt in Wasser; INCI-Bezeichnung: Acrylamidopropyltrimonium Chloride/Acrylates Copolymer (Stockhausen) |
| Cremophor^{®} A 25 | C₁₆₋₁₈-Fettalkohol ethoxyliert mit 25 Einheiten Ethylenoxid (INCI-Bezeichnung: Ceteareth-25) (BASF) |

### 1. Wirksamkeit

### 1.1 Bereitstellung der Testformulierungen

Folgende Rezepturen wurden bereitgestellt. Alle Mengenangaben sind soweit nicht anders gekennzeichnet Gew.-%.

**Tabelle 1:**

| | T-0 (Vergleich) | T-1 (erfindungsgemäß) |
|---|---|---|
| Hydrenol^{®} D | 3,40 g | 3,40 g |
| Lorol^{®} techn. | 1,40 q | 1,40 g |
| Dehyton^{®} K | 4,50 g | 4,50 g |
| Ölsäure | 0,24 g | 0,24 g |
| Eumulgin^{®} B2 | 0,20 g | 0,20 g |
| 2-Aminoethan-1-ol | 3,20 g | 3,20 g |
| L-Arqinin | 0,50 g | 0,50 g |
| Turpinal SL | 0,10 g | 0,10 g |
| Natronwasserglas 40/42 | 0,20 g | 0,20 g |
| Natronlauge (45 %iq) | 0,30 g | 0,30 g |
| Dipicolinsäure | 0,05 g | 0,05 g |
| Dow Corning DB 110 A | 0,03 g | 0,03 g |
| Aculyn 33 | 7,10 | 7,10 |
| Kaliumpersulfat | 7,40 | 7,40 |
| Kieselsäure | 0,10 | 0,10 |
| Linolsäure | - | 0,75 |
| Linolensäure | - | 0,01 |
| Wasserstoffperoxid | 5,3 | 5,3 |
| Wasser | 55,5 | 54,5 |

### 1.2 Haarbehandlung

Die Zusammensetzungen T-0 (ohne erfindungsgemäßes Gemisch) und T-1 (mit erfindungsgemäßem Gemisch) wurden jeweils direkt nach ihrer Herstellung im Gewichtsverhältnis zum Gewicht des Testhaars von 4 zu 1 für 30 min bei 32°C auf Haarsträhnen (Kerling Euro-Naturhaar 6/0) appliziert. Die Haarsträhnen wurden anschließend mit Wasser gespült und getrocknet. Der Vorgang wurde 1x wiederholt.

### 1.3 Ermittlung des Haarschädigungsgrads

Die an den Testhaarsträhnen durch die Haarbehandlung aus Punkt 1.2 hervorgerufene Haarschädigung wurde durch die Messung des Kontaktwinkels nach Wilhelmy quantifiziert.

Diese Methode wurde wie in der Literatur beschrieben durchgeführt (Molina R et al: Chemical modifications on human hair studies by means of contact angle determination. J. Coll. Int. Sci., 2001, 237, 40-46).

Der Kontaktwinkel der unbehandelten Haare lag bei 103,7° +/- 2,4° (Messwert +/-Standardabweichung, 10 Messungen) und sank nach der unter 1.2 beschriebenen 2-maligen Behandlung mit T-0 auf einen mittleren Wert von 63,4° +/- 4,7° (Messwert +/-Standardabweichung, 10 Messungen). Damit zeigt sich erwartungsgemäß eine verminderte Hydrophobizität. Nach der unter 1.2 beschriebenen 2-maligen Behandlung mit dem erfindungsgemäßen Mittel T-1 wurde ein Wert von 68,4° +/- 4,7° (Messwert +/-Standardabweichung, 10 Messungen) gemessen. Dies zeigt, dass die Haaroberfläche durch Einsatz des Gemischs von Linolsäure und Linolensäure vor den Folgen der oxidativen Haarbehandlung geschützt wird.

### 2. Beispielrezepturen .

Die Mengenangaben sind, falls nicht anders gekennzeichnet, Gew.-% bezogen auf das Gesamtgewicht der jeweiligen Rezeptur.

### 2.1 Blondiermittel

Die Blondiermittel B1, B2 und B3 bestehen jeweils aus einer Blondiercreme und einem dazugehörigen Entwickler, sowie einem Bleichverstärker. Zur Herstellung des anwendungsbereiten Blondiermittels wurden 50 g des Bleichverstärkers in 50 g der entsprechenden Blondiercreme gegeben. Unter Rühren wurden 20 g des entsprechenden Entwicklers zugegeben. Bei Applikation auf Kopfhaar und einer Einwirkungszeit von 30 Minuten wurde nach dem Spülen und Trocknen eine hervorragende Aufhellung beobachtet.

### 2.1.1 Blondiercreme

| | B1 | B2 | B3 |
|---|---|---|---|
| Eumulgin B1 | 0,60 | - | - |
| Eumulgin B2 | 0,60 | 0,50 | - |
| Hydrenol D | 6,90 | 7,50 | 12,00 |
| Lorol techn. | 2,50 | 3,00 | 2,50 |
| Texapon NSO UP | 7,50 | 16,00 | 26,00 |
| Stabylen 30 | - | - | 0,10 |
| Cetiol OE | - | - | 0,20 |
| Dehyton K | - | 10,00 | - |
| 2-Aminoethan-1-ol | - | 8,00 | - |
| L-Arginin | - | 1,00 | - |
| Turpinal SL | 0,20 | 0,20 | 0,20 |
| Natronwasserglas 40/42 | 0,50 | 0,50 | 0,50 |
| Akypo Soft 45 NV | 8,00 | - | - |
| Plantacare 1200 UP | 1,50 | - | - |
| Polymer W 37194 | 3,80 | - | - |
| Ammoniumsulfat | 1,00 | - | 1,00 |
| Ascorbinsäure | 0,10 | - | - |
| Kaliumhydroxid | 0,80 | - | - |
| Ammoniak (25 %ige wässrige Lösunq) | 7,10 | - | 7,60 |
| Gluadin W 40 | - | - | 0,40 |
| Linolsäure | 0,75 | 0,75 | 0,75 |
| Linolensäure | 0,01 | 0,01 | 0,01 |
| Wasser | ad. 100 | ad. 100 | ad. 100 |

### 2.1.2 Entwickler

| | B1 | B2 | B3 |
|---|---|---|---|
| Lorol techn. | 3,60 | 3,60 | 3,60 |
| Eumulgin B2 | 0,90 | 0,90 | 0,90 |
| Disponil FES 77 IS | 2,25 | 2,25 | 2,25 |
| Wasserstoffperoxid 50% | 24,0 | 24,0 | 24,0 |
| Turpinal SL | 1,50 | 1,50 | 1,50 |
| Aculyn 33A | 15,0 | 15,0 | 15,0 |
| Wasser | ad. 100 | ad. 100 | ad. 100 |

### 2.1.3 Bleichverstärker

| | |
|---|---|
| (NH₄)₂S₂O₈ | 98,6 |
| Aerosil 200 | 1,4 |

### 2.2 oxidative Färbemittel

Die Färbemittel F1 bis F16 bestehen jeweils aus einer Färbecreme und einem dazugehörigen Entwickler. Zur Herstellung des anwendungsbereiten Färbemittels wurden 50 g der Färbecreme unter Rühren mit 50 g des entsprechenden Entwicklers gemischt. Bei Applikation auf Kopfhaar und einer Einwirkungszeit von 30 Minuten wurde nach dem Spülen und Trocknen ein hervorragendes Färbeergebnis beobachtet.

### 2.2.1 Färbecremes

| | F1 | F2 | F3 | F4 | F5 | F6 | F7 | F8 |
|---|---|---|---|---|---|---|---|---|
| Hydrenol D | 5,50 | 5,50 | 5,50 | 5,50 | 5,50 | 5,50 | 5,50 | 5,50 |
| Lorol 16 | 2,00 | 2,00 | 2,00 | 2,00 | 2,00 | 2,00 | 2,00 | 2,00 |
| Eumulgin B1 | 0,50 | 0,50 | 0,50 | 0,50 | 0,50 | 0,50 | 0,50 | 0,50 |
| Eumulgin B2 | 0,50 | 0,50 | 0,50 | 0,50 | 0,50 | 0,50 | 0,50 | 0,50 |
| Lamesoft PO 65 | 2,00 | 2,00 | 2,00 | 2,00 | 2,00 | 2,00 | 2,00 | 2,00 |
| Akypo Soft 45 NV | 10,00 | 10,00 | 10,00 | 10,00 | 10,00 | 10,00 | 10,00 | 10,00 |
| Texapon K 14 S 70% | 2,80 | 2,80 | 2,80 | 2,80 | 2,80 | 2,80 | 2,80 | 2,80 |
| Ammoniak 25% | 6,50 | 6,50 | 6,50 | 6,50 | 6,50 | 6,50 | 6,50 | 6,50 |
| Turpinal SL | 0,20 | 0,20 | 0,20 | 0,20 | 0,20 | 0,20 | 0,20 | 0,20 |
| p-Toluylendiamisulfat | 2,50 | 0,40 | 0,30 | 0,05 | 0,30 | 2,50 | 0,40 | 0,30 |
| Resorcin | 0,40 | 0,10 | 1,00 | 0,02 | 0,03 | 0,40 | 0,10 | 1,00 |
| 2,4-Diaminophenoxyethanol 2HCl | 2,00 | - | - | - | - | 2,00 | - | - |
| 2-Methylresorcin | - | 1,00 | 0,03 | - | 0,50 | - | 1,00 | 0,03 |
| 2,4,5,6-Tetraaminopyrimidinsulfat | - | 1,50 | - | - | - | - | 1,50 | - |
| Bis-(5-Amino-2-hydroxyphenyl)methan 2HCl | - | 0,06 | - | - | - | - | 0,06 | - |
| m-Aminophenol | - | - | 0,01 | 0,03 | 0,02 | - | - | 0,01 |
| 4-Chlorresorcin | - | - | 0,60 | 0,01 | 0,10 | - | - | 0,60 |
| Ascorbinsäure | 0,10 | 0,10 | 0,10 | 0,10 | 0,10 | 0,10 | 0,10 | 0,10 |
| Natriumsulfit 96% | 0,40 | 0,40 | 0,40 | 0,40 | 0,40 | 0,40 | 0,40 | 0,40 |
| Linolsäure | 0,75 | 0,75 | 0,75 | 0,75 | 0,75 | 1,00 | 0,75 | 0,75 |
| Linolensäure | 0,01 | 0,01 | 0,01 | 0,01 | 0,01 | 0,02 | 0,01 | 0,01 |
| Aqua | < -------------------------- ad. 100 ------------------------ > | | | | | | | |

| | F9 | F10 | F11 | F12 | F13 | F14 | F15 | F16 |
|---|---|---|---|---|---|---|---|---|
| Cetearyl Alkohol | 8,00 | 8,00 | 8,00 | 8,00 | 8,00 | 8,00 | 8,00 | 8,00 |
| Lorol | 3,00 | 3,00 | 3,00 | 3,00 | 3,00 | 3,00 | 3,00 | 3,00 |
| Eumulqin B2 | 2,00 | 2,00 | 2,00 | 2,00 | 2,00 | 2,00 | 2,00 | 2,00 |
| Ascorbinsäure | 0,50 | 0,50 | 0,50 | 0,50 | 0,50 | 0,50 | 0,50 | 0,50 |
| Natriumsulfit 96% | 0,50 | 0,50 | 0,50 | 0,50 | 0,50 | 0,50 | 0,50 | 0,50 |
| Turpinal SL | 0,20 | 0,20 | 0,20 | 0,20 | 0,20 | 0,20 | 0,20 | 0,20 |
| Gluadin W 40 | 4,00 | 4,00 | 4,00 | 4,00 | 4,00 | 4,00 | 4,00 | 4,00 |
| Ammoniak 25% | 6,00 | 6,00 | 6,00 | 6,00 | 6,00 | 6,00 | 6,00 | 6,00 |
| Resorcin | 0,10 | 8,00 | 8,00 | 8,00 | 8,00 | 8,00 | 8,00 | 8,00 |
| p-Toluylendiaminsulfat | 0,15 | 0,70 | 1,80 | 0,15 | 0,70 | 1,80 | 0,70 | 1,80 |
| 4-Chlorresorcin | 0,03 | 0,02 | 0,50 | 0,03 | 0,02 | 0,50 | 0,02 | 0,50 |
| 2-Amino-2-metylamino-6-methoxypyridin | 0,01 | - | - | 0,01 | - | - | - | - |
| 2,6-Dihydroxy-3,4-Dimethylpyridin | - | 0,02 | - | - | 0,02 | - | 0,02 | - |
| 2,7-Dihydroxynaphthalin | - | 0,01 | - | - | 0,01 | - | 0,01 | - |
| 2-Methylresorcin | - | 0,05 | - | - | 0,05 | - | 0,05 | - |
| m-Aminophenol | - | 0,10 | - | - | 0,10 | - | 0,10 | - |
| 2,4-Diaminophenoxyethanol 2 HCl | - | 0,01 | - | - | 0,01 | - | 0,01 | - |
| 3-Amino-2-methylamino-6-methoxypyridin | - | 0,01 | 0,10 | - | 0,01 | 0,10 | 0,01 | 0,10 |
| 1,3-Bis-(2,4-diaminophenoxy)propan 4HCl | - | - | 0,30 | - | - | 0,30 | - | 0,30 |
| Linolsäure | 0,75 | 0,75 | 0,75 | 0,75 | 1,50 | 0,75 | 0,75 | 0,75 |
| Linolensäure | 0,01 | 0,01 | 0,01 | 0,01 | 0,04 | 0,01 | 0,01 | 0,01 |
| Wasser | < --------------------------- ad. 100 --------------------------- > | | | | | | | |

### 2.2.2 Entwickler

| | F1 | F2 | F3 | F4 | F5 | F6 | F7 | F8 |
|---|---|---|---|---|---|---|---|---|
| Dipicolinsäure | 0,10 | 0,10 | 0,10 | 0,10 | 0,10 | 0,10 | 0,10 | 0,10 |
| Dinatriumpyrophosphat | 0,03 | 0,03 | 0,03 | 0,03 | 0,03 | 0,03 | 0,03 | 0,03 |
| Turpinal SL | 1,50 | 1,50 | 1,50 | 1,50 | 1,50 | 1,50 | 1,50 | 1,50 |
| Texapon NSO UP | 2,00 | 2,00 | 2,00 | 2,00 | 2,00 | 2,00 | 2,00 | 2,00 |
| Aculyn 33A | 12,00 | 12,00 | 12,00 | 12,00 | 12,00 | 12,00 | 12,00 | 12,00 |
| Wasserstoffperoxid 50% | 12,00 | 6,00 | 12,00 | 6,00 | | 12,00 6,00 | 12,00 | 6,00 |
| Ammoniak | 0,62 | 0,62 | 0,62 | 0,62 | 0,62 | 0,62 | 0,62 | 0,62 |
| Wasser | < ------------------------ ad. 100 ----------------------------> | | | | | | | |

| | F9 | F10 | F11 | F12 | F13 | F14 | F15 | F16 |
|---|---|---|---|---|---|---|---|---|
| Lorol C16 | 3,50 | 3,50 | 3,50 | 3,50 | 3,50 | 3,50 | 3,50 | 3,50 |
| Eumulgin B2 | 1,00 | 1,00 | 1,00 | 1,00 | 1,00 | 1,00 | 1,00 | 1,00 |
| Disponil FES 77 IS | 2,50 | 2,50 | 2,50 | 2,50 | 2,50 | 2,50 | 2,50 | 2,50 |
| Wasserstoffperoxid 50% | 24,00 | 20,00 | 12,00 | 24,00 | 20,00 | 12,00 | 24,00 | 20,00 |
| Turpinal SL | 1,50 | 1,50 | 1,50 | 1,50 | 1,50 | 1,50 | 1,50 | 1,50 |
| Aculyn 33A | 10,0 | 10,0 | 10,0 | 10,0 | 10,0 | 10,0 | 10,0 | 10,0 |
| Wasser | < ------------------------ ad. 100 --------------------- > | | | | | | | |

### 2.3 ammoniakfreie oxidative Färbemittel

Die Färbemittel F17 bis F25 bestehen jeweils aus einer Färbecreme und dem nachfolgend genannten Entwickler. Zur Herstellung des anwendungsbereiten Färbemittels wurden 50 g der Färbecreme unter Rühren mit 50 g des Entwicklers gemischt. Bei Applikation auf Kopfhaar und einer Einwirkungszeit von 30 Minuten wurde nach dem Spülen und Trocknen ein hervorragendes Färbeergebnis beobachtet.

### 2.3.1 Färbecremes

| | F17 | F18 | F19 | F20 | F21 | F22 | F23 | F24 | F25 |
|---|---|---|---|---|---|---|---|---|---|
| Hydrenol D | 7,15 | 7,15 | 7,15 | 7,15 | 7,15 | 7,15 | 7,15 | 7,15 | 7,15 |
| Lorol | 2,60 | 2,60 | 2,60 | 2,60 | 2,60 | 2,60 | 2,60 | 2,60 | 2,60 |
| Eumulgin B1 | 0,65 | 0,65 | 0,65 | 0,65 | 0,65 | 0,65 | 0,65 | 0,65 | 0,65 |
| Eumulgin B2 | 0,65 | 0,65 | 0,65 | 0,65 | 0,65 | 0,65 | 0,65 | 0,65 | 0,65 |
| Akypo Soft 45 NV | 10,0 | 10,0 | 10,0 | 10,0 | 10,0 | 10,0 | 10,0 | 10,0 | 10,0 |
| Texapon K 14 S 70% | 2,80 | 2,80 | 2,80 | 2,80 | 2,80 | 2,80 | 2,80 | 2,80 | 2,80 |
| Plantacare 1200 | 2,00 | 2,00 | 2,00 | 2,00 | 2,00 | 2,00 | 2,00 | 2,00 | 2,00 |
| Produkt W 37194 | 3,75 | 3,75 | 3,75 | 3,75 | 3,75 | 3,75 | 3,75 | 3,75 | 3,75 |
| 2-Aminoethan-1-ol | 3,10 | 2,80 | 2,00 | 3,10 | 2,80 | 2,00 | 3,10 | 2,80 | 2,00 |
| L-Arqinin | - | 1,00 | - | - | 1,00 | - | - | 1,00 | - |
| 2-Amino-2-metylpropanol | - | - | 1,50 | - | - | 1,50 | - | - | 1,50 |
| Natriumchlorid | 0,50 | 0,50 | 0,50 | 0,50 | 0,50 | 0,50 | 0,50 | 0,50 | 0,50 |
| Natriumsulfit | 0,40 | 0,40 | 0,40 | 0,40 | 0,40 | 0,40 | 0,40 | 0,40 | 0,40 |
| Ascorbinsäure | 0,10 | 0,10 | 0,10 | 0,10 | 0,10 | 0,10 | 0,10 | 0,10 | 0,10 |
| Turpinal SL | 0,20 | 0,20 | 0,20 | 0,20 | 0,20 | 0,20 | 0,20 | 0,20 | 0,20 |
| Natronwasserglas 40/42 | 0,50 | 0,50 | 0,50 | 0,50 | 0,50 | 0,50 | 0,50 | 0,50 | 0,50 |
| Kaliumhydroxid 50% | 1,00 | 1,00 | 1,00 | 1,00 | 1,00 | 1,00 | 1,00 | 1,00 | 1,00 |
| Glycin | 1,00 | 1,00 | 1,00 | 1,00 | 1,00 | 1,00 | 1,00 | 1,00 | 1,00 |
| Linolsäure | 0,75 | 0,75 | 1,50 | 0,75 | 1,50 | 0,75 | 0,75 | 0,75 | 0,75 |
| Linolensäure | 0,01 | 0,01 | 0,03 | 0,01 | 0,02 | 0,01 | 0,01 | 0,01 | 0,01 |
| p-Toluylendiamisulfat | 0,40 | 0,40 | 0,40 | 0,40 | 0,40 | 0,40 | 0,40 | 0,40 | 0,40 |
| 2,7-Dihydroxynaphtalin | 0,03 | 0,03 | 0,03 | 0,03 | 0,03 | 0,03 | 0,03 | 0,03 | 0,03 |
| Resorcin | 0,12 | 0,12 | 0,12 | 0,12 | 0,12 | 0,12 | 0,12 | 0,12 | 0,12 |
| 4-Chlorresorcin | 0,06 | 0,06 | 0,06 | 0,06 | 0,06 | 0,06 | 0,06 | 0,06 | 0,06 |
| 2-Methylresorcin | 0,02 | 0,02 | 0,02 | 0,02 | 0,02 | 0,02 | 0,02 | 0,02 | 0,02 |
| m-Aminophenol | 0,02 | 0,02 | 0,02 | 0,02 | 0,02 | 0,02 | 0,02 | 0,02 | 0,02 |
| Wasser | < ----------------------- ad 100 ---------------------- > | | | | | | | | |

### 2.3.2 Entwickler

| | |
|---|---|
| Dipicolinsäure | 0,10 |
| Dinatriumpyrophosphat | 0,03 |
| Turpinal SL | 1,50 |
| Texapon NSO UP | 2,00 |
| Aculyn 33A | 12,00 |
| Wasserstoffperoxid 50% | 12,00 |
| Wasser | ad. 100 |

## Patentansprüche

1. Verwendung eines Gemisches aus Linolsäure und Linolensäure in einem Gewichts-verhältnisbereich von Linolsäure zu Linolensäure von 1 zu 0.0005 bis 1 zu 0.1 zur Verminderung der oxidativen Haarschädigung im Rahmen einer oxidativen Haarbehandlung.

2. Mittel, enthaltend in einem kosmetischen Träger eine Kombination aus
(i) mindestens einem Oxidationsmittel und
(ii) einem Gemisch aus Linolsäure und Linolensäure,
**dadurch gekennzeichnet, dass** die Linolsäure und die Linolensäure in einem Gewichtsverhältnisbereich von Linolsäure zu Linolensäure von 1 zu 0.0005 bis 1 zu 0.1 enthalten sind.

3. Mittel nach Anspruch 2, **dadurch gekennzeichnet, daß** es die Linolsäure in einer Menge von 0,01 Gew.% bis 10 Gew.-%, insbesondere von 0,1 Gew.% bis 1 Gew.-%, enthält, wobei die Mengen jeweils auf das Gewicht des Mittels bezogen werden.

4. Mittel nach einem der Ansprüche 2 oder 3, **dadurch gekennzeichnet, dass** die Linolensäure in einer Menge von 0,0001 Gew.-% bis 1 Gew.%, insbesondere von 0,01 Gew.% bis 0,1 Gew.-%, enthaiten ist, wobei die Mengen jeweils auf das Gewicht des Mittels bezogen werden. ist

5. Mittel nach einem der Ansprüche 2 bis 4, **dadurch gekennzeichnet, dass** die Linolsäure und die Linolensäure in einem Gewichtsverhältnisbereich von Linolsäure zu Linolensäure von 1 zu 0,001 bis 1 zu 0,05-enthalten sind.

6. Mittel nach einem der Ansprüche 2 bis 5, **dadurch gekennzeichnet, daß** es zusätzlich mindestens eine farbverändernde Komponente enthält.

7. Mittel nach Anspruch 6, **dadurch gekennzeichnet, daß** die farbverändernde Komponente ausgewählt wird
(a) aus mindestens einem Oxidationsfarbstoffvorprodukt vom Typ der Entwicklerkomponenten und gegebenenfalls zusätzlich mindestens einer Kupplerkomponente
und/oder
(b) aus Oxofarbstoffvorprodukten
und/oder
(c) aus mindestens einem direktziehenden Farbstoff
und/oder
(d) aus mindestens einer Vorstufe naturanaloger Farbstoffe
und/oder
(e) aus mindestens einem Bleichverstärker.

8. Mittel nach einem der Ansprüche 2 bis 7, **dadurch gekennzeichnet, daß** es zusätzlich mindestens ein Tensid enthält.

9. Mittel nach einem der Ansprüche 2 bis 8, **dadurch gekennzeichnet, daß** das Oxidationsmittel Wasserstoffperoxyd und/oder mindestens ein Anlagerungsprodukt von Wasserstoffperoxyd an anorganische oder organische Verbindungen ist.

10. Mittel nach einem der Ansprüche 2 bis 9, **dadurch gekennzeichnet, daß** das Oxidationsmittel in einer Menge von 1,0 bis 10,0 Gew.% bezogen auf das Gewicht des anwendungsbereiten Mittels, enthalten ist.

11. Verfahren zur oxidativen Haarbehandlung, in dem ein oxidatives kosmetisches Mittel, enthaltend in einem kosmetischen Träger mindestens ein Oxidationsmittel auf das Haar aufgetragen wird und nach einer Einwirkungszeit vom Haar gespült wird, **dadurch gekennzeichnet, daß**
- unmittelbar vor dem Auftragen des oxidativen kosmetischen Mittels ein kosmetisches Mittel, enthaltend in einem kosmetischen Träger ein Gemisch aus Linolsäure und Linolensäure in einem Gewichtsverhältnisbereich von Linolsäure zu Linolensäure von 1 zu 0.0005 bis 1 zu 0.1, aufgetragen und gegebenenfalls nach einer Einwirkungszeit wieder abgespült wird
und/oder
- das oxidative kosmetische Mittel zusätzlich ein Gemisch aus Linolsäure und Linolensäure in einem Gewichtsverhältnisbereich von Linolsäure zu Linolensäure von 1 zu 0.0005 bis 1 zu 0.1, enthält.

12. Verpackungseinheit, umfassend in einem ersten Kontainer ein oxidatives kosmetisches Mittel, enthaltend in einem kosmetischen Träger mindestens ein Oxidationsmittel und in einem zweiten Kontainer ein kosmetische Mittel, enthaltend in einem kosmetischen Träger ein Gemisch aus Linolsäure und Linolensäure in einem Gewichtsverhältnisbereich von Linolsäure zu Linolensäure von 1 zu 0.0005 bis 1 zu 0.1.

## Claims

1. Use of a mixture of linoleic acid and linolenic acid in a weight ratio range of linoleic acid to linolenic acid of 1:0.0005 to 1:0.1 for reducing oxidative hair damage in the context of oxidative hair treatment.

2. An agent containing in a cosmetic carrier a combination of
(i) at least one oxidising agent and
(ii) a mixture of linoleic acid and linolenic acid,
**characterised in that** the linoleic acid and the linolenic acid are present in a weight ratio range of linoleic acid to linolenic acid of 1:0.0005 to 1:0.1.

3. An agent according to claim 2, **characterised in that** it contains the linoleic acid in a quantity of 0.01 wt.% to 10 wt.%, in particular of 0.1 wt.% to 1 wt.%, wherein the quantities in each case relate to the weight of the agent.

4. An agent according to either one of claims 2 or 3, **characterised in that** the linolenic acid is present in a quantity of 0.0001 wt.% to 1 wt.%. in particular of 0.01 wt.% to 0.1 wt.%, wherein the quantities in each case relate to the weight of the agent.

5. An agent according to any one of claims 2 to 4, **characterised in that** the linoleic acid and the linolenic acid are present in a weight ratio range of linoleic acid to linolenic acid of 1:0.001 to 1:0.05.

6. An agent according to any one of claims 2 to 5, **characterised in that** it additionally contains at least one colour-modifying component.

7. An agent according to claim 6, **characterised in that** the colour-modifying component is selected
(a) from at least one oxidation dye precursor of the developer component type and optionally additionally at least one coupler component
and/or
(b) from oxo dye precursors and/or
(c) from at least one direct dye
and/or
(d) from at least one precursor of nature-analogous dyes and/or
(e) from at least one bleach booster.

8. An agent according to any one of claims 2 to 7, **characterised in that** it additionally contains at least one surfactant.

9. An agent according to any one of claims 2 to 8, **characterised in that** the oxidising agent is hydrogen peroxide and/or at least one addition product of hydrogen peroxide onto inorganic or organic compounds.

10. An agent according to any one of claims 2 to 9, **characterised in that** the oxidising agent is present in a quantity of 1.0 to 10.0 wt.% relative to the weight of the ready-to-use agent.

11. A method for oxidative hair treatment, in which an oxidative cosmetic agent containing at least one oxidising agent in a cosmetic carrier is applied onto the hair and, after a period of exposure, is rinsed out of the hair, **characterised in that**,
- immediately prior to application of the oxidative cosmetic agent, a cosmetic agent containing in a cosmetic carrier a mixture of linoleic acid and linolenic acid in a weight ratio range of linoleic acid to linolenic acid of 1:0.0005 to 1:0.1 is applied and, after a period of exposure, if necessary rinsed back out
and/or
- the oxidative cosmetic agent additionally contains a mixture of linoleic acid and linolenic acid in a weight ratio range of linoleic acid to linolenic acid of 1:0.0005 to 1:0.1.

12. A packaging unit comprising in a first container an oxidative cosmetic agent containing in a cosmetic carrier at least one oxidising agent and in a second container a cosmetic agent containing in a cosmetic carrier a mixture of linoleic acid and linolenic acid in a weight ratio range of linoleic acid to linolenic acid of 1:0.0005 to 1:0.1.

## Revendications

1. Utilisation d'un mélange d'acide linoléique et d'acide linolénique, le rapport pondéral entre l'acide linoléique et l'acide linolénique étant compris entre 1 pour 0,0005 et 1 pour 0,1, pour réduire les dommages oxydatifs que les cheveux subissent dans le cadre d'un traitement capillaire oxydatif.

2. Produit, contenant dans un support cosmétique une combinaison constituée
(I) d'au moins un agent oxydant et
(II) d'un mélange d'acide linoléique et d'acide linolénique,
**caractérisé en ce qu'**il contient l'acide linoléique et l'acide linolénique dans un rapport pondéral entre l'acide linoléique et acide linolénique compris entre 1 pour 0,0005 et 1 pour 0,1.

3. Produit selon la revendication 2, **caractérisé en ce qu'**il contient l'acide linoléique dans une quantité comprise entre 0,01 % en poids et 10 % en poids, notamment entre 0,1 % en poids et 1 % en poids, les quantités étant toujours exprimées par rapport au poids dudit produit.

4. Produit selon l'une des revendications 2 ou 3, **caractérisé en ce qu'**il contient l'acide linolénique dans une quantité comprise entre 0,0001 % en poids et 1 % en poids, notamment entre 0,01 % en poids et 0,1 % en poids, les quantités étant toujours exprimées par rapport au poids dudit produit.

5. Produit selon l'une des revendications 2 à 4, **caractérisé en ce qu'**il contient l'acide linoléique et l'acide linolénique dans un rapport pondéral entre l'acide linoléique et acide linolénique compris entre 1 pour 0,001 et 1 pour 0,05.

6. Produit selon l'une des revendications 2 à 5, **caractérisé en ce qu'**il contient en outre au moins un composant apte à modifier la couleur.

7. Produit selon la revendication 6, **caractérisé en ce que** le composant apte à modifier la couleur est choisi parmi
(a) au moins un précurseur de colorant d'oxydation appartenant à la catégorie des bases d'oxydation, un coupleur lui étant éventuellement associé.
et/ou
(b) les précurseurs de colorants à groupe oxo
et/ou
(c) au moins un colorant direct
et/ou
(d) au moins un précurseur de colorants analogues à des produits naturels
et/ou
(e) au moins un agent renforçateur de blanchiment.

8. Produit selon l'une des revendications 2 à 7, **caractérisé en ce qu'**il contient en outre au moins un agent tensioactif.

9. Produit selon l'une des revendications 2 à 8, **caractérisé en ce que** ledit agent oxydant est du peroxyde d'hydrogène et/ou au moins un produit résultant de l'addition de peroxyde d'hydrogène à des composés inorganiques ou organiques.

10. Produit selon l'une des revendications 2 à 9, **caractérisé en ce qu'**il contient ledit agent oxydant dans une quantité de 1,0 à 10,0 % en poids, par rapport au poids du produit prêt à l'emploi.

11. Procédé de traitement capillaire oxydatif consistant à appliquer sur les cheveux un produit cosmétique oxydatif contenant au moins un agent oxydant dans un support cosmétique puis à enlever ledit produit au terme d'un délai d'action en rinçant les cheveux, **caractérisé en ce que**,
- immédiatement avant l'application de l'agent cosmétique oxydatif, on applique un agent cosmétique contenant dans un support cosmétique un mélange d'acide linoléique et d'acide linolénique dans un rapport pondéral entre l'acide linoléique et acide linolénique compris entre 1 pour 0,0005 et 1 pour 0,1, pour l'enlever éventuellement au terme d'un délai d'action en effectuant un rinçage et/ou
- le produit cosmétique oxydatif contient en outre un mélange d'acide linoléique et d'acide linolénique dans un rapport pondéral entre l'acide linoléique et acide linolénique compris entre 1 pour 0,0005 et 1 pour 0,1.

12. Unité d'emballage comprenant, dans un premier récipient, un produit cosmétique oxydatif contenant dans un support cosmétique au moins un agent oxydant et, dans un deuxième récipient, un produit cosmétique contenant dans un support cosmétique un mélange d'acide linoléique et d'acide linolénique dans un rapport pondéral entre l'acide linoléique et acide linolénique compris entre 1 pour 0,0005 et 1 pour 0,1.
